# EUROPÄISCHE PATENTSCHRIFT

(11) **EP 2 204 445 B1**
(45) Veröffentlichungstag und Bekanntmachung des Hinweises auf die Patenterteilung: **02.01.2013**
(21) Anmeldenummer: 10158113.0
(22) Anmeldetag: 25.07.2006
(51) Int. Cl.: C12N 9/12, C12Q 1/68

(54) **Verfahren und Kit zur primer-abhängigen Amplifikation heterologer viraler, eukaryontischer oder prokaryontischer RNA**
Methods and kit for primer-dependent amplification heterologous viral, eukaryotic and prokaryotic RNA
Procede et kit pour l'amplification primer-dependent de ARN heterologue viral, eucaryoteque et procaryotique.

(30) Priorität: 25.07.2005 DE 102005036085; 13.02.2006 DE 102006008219
(43) Veröffentlichungstag der Anmeldung: 07.07.2010
(62) Teilanmeldung aus: 09155974.0
(73) Patentinhaber: RiboxX GmbH, 01445 Radebeul (DE)
(72) Erfinder: Rohayem, Jacques, 01277 Dresden (DE); Rudolph, Wolfram, 01307 Dresden (DE); Jäger, Katrin, 01462 Dresden (DE); Jacobs, Enno, 01309 Dresden (DE)
(74) Vertreter: Schnabel, Jörg

(56) Entgegenhaltungen:
- WO-A2-02/092774
- SEAH E.L. ET AL.,: "open reading frame 1 of the norwalk-like virus camberwell: completion of sequence and expression in mammalian cells" J. VIROLOGY, Bd. 73, Nr. 12, Dezember 1999 (1999-12), Seiten 10531-10535, XP002418059
- WANG J. ET AL.,: "sequence diversity of small, round-structured viruses in the norwalk virus group" J. VIROLOGY, Bd. 68, Nr. 9, August 1994 (1994-08), Seiten 5982-5990, XP002418060
- GUO M. ET AL.,: "molecular characterization of porcine enteric calicivirus genetically related to sapporo-like human caliciviruses" J. VIROLOGY, Bd. 73, Nr. 11, November 1999 (1999-11), Seiten 9625-9631, XP002418061
- BELLIOT GAEL ET AL: "Norovirus proteinase-polymerase and polymerase are both active forms of RNA-dependent RNA polymerase" JOURNAL OF VIROLOGY, Bd. 79, Nr. 4, Februar 2005 (2005-02), Seiten 2393-2403, XP002581907 ISSN: 0022-538X
- WEI LAI ET AL: "Proteinase-polymerase precursor as the active form of feline calicivirus RNA-dependent RNA polymerase" JOURNAL OF VIROLOGY, Bd. 75, Nr. 3, Februar 2001 (2001-02), Seiten 1211-1219, XP002581908 ISSN: 0022-538X
- VAZQUEZ ANA LOPEZ ET AL: "Expression of enzymatically active rabbit hemorrhagic disease virus RNA-dependent RNA polymerase in Escherichia coli" JOURNAL OF VIROLOGY, Bd. 72, Nr. 4, April 1998 (1998-04), Seiten 2999-3004, XP002581909 ISSN: 0022-538X

### SEQUENCE LISTING

<110> Technische Universität Dresden
<120> Verfahren und Kit zur primer-abhängigen Amplifikation heterologer viraler, eukaryontischer oder prokaryontischer RNA
<130> T 0030 WOEPT1T1
<150> DE 10 2005 036 085.8
   <151> 2005-07-25
<150> DE 10 2006 008 219.2
   <151> 2006-02-13
<160> 6
<170> PatentIn version 3.3
<210> 1
   <211> 520
   <212> PRT
   <213> Norovirus
<400> 1

Met Gly Gly Asp Ser Lys Gly Thr Tyr Cys Gly Ala Pro Ile Leu Gly
1               5                   10                  15

Pro Gly Ser Ala Pro Lys Leu Ser Thr Lys Thr Lys Phe Trp Arg Ser
            20                  25                  30

Ser Thr Thr Pro Leu Pro Pro Gly Thr Tyr Glu Pro Ala Tyr Leu Gly
        35                  40                  45

Gly Lys Asp Pro Arg Val Lys Gly Gly Pro Ser Leu Gln Gln Val Met
    50                  55                  60

Arg Asp Gln Leu Lys Pro Phe Thr Glu Pro Arg Gly Lys Pro Pro Lys
65                  70                  75                  80

Pro Ser Val Leu Glu Ala Ala Lys Lys Thr Ile Ile Asn Val Leu Glu
                85                  90                  95

Gln Thr Ile Asp Pro Pro Glu Lys Trp Ser Phe Thr Gln Ala Cys Ala
            100                 105                 110

Ser Leu Asp Lys Thr Thr Ser Ser Gly His Pro His His Met Arg Lys
        115                 120                 125

Asn Asp Cys Trp Asn Gly Glu Ser Phe Thr Gly Lys Leu Ala Asp Gln
    130                 135                 140

Ala Ser Lys Ala Asn Leu Met Phe Glu Gly Gly Lys Asn Met Thr Pro

## Beschreibung

Die Erfindung betrifft ein Verfahren und ein Kit zur primer-abhängigen Amplifikation von heterologer viraler, eukaryontischer oder prokaryontischer Ribonukleinsäure (RNA) gemäß Anspruch 1 bzw. 13. Das erfindungsgemäße Amplifikationsverfahren eignet sich insbesondere für die Microarray-Technologie und zur Diagnose viraler Infektionen durch den Nachweis viraler RNA in Patientenmaterial.

Die RNA ist ein wichtiger Bestandteil der eukaryontischen und der prokaryontischen Zelle. Sie ist an mehreren vitalen Funktionen beteiligt: der Transkription, d.h. der Umschreibung der Information des Erbgutes (Desoxyribonukleinsäure, DNA), der Übertragung dieser Information vom Zellkern in das Zytoplasma (mRNA), und der Translation, d.h. die Übersetzung der umgeschriebenen Information In Aminosäuren bzw. Proteine. Die RNA bildet auch die sog. Transfer-RNA, einen wichtigen Baustein für die Translation. Die RNA ist auch ein wichtiger Bestandteil der Ribosomen. Dies sind strukturelle Einheiten des Zytoplasmas, an denen die Übersetzung der Information in Eiweiß stattfindet.

Die Bedeutung der RNA im Bereich der Biotechnologie hat in den letzten Jahrzehnten zugenommen. Neue Entwicklungen wie z.B. die Microarray-Technologie, die miRNA (micro-RNA) oder die siRNA-Technologie (small interfering RNA) haben stattgefunden, deren Relevanz im Bereich der Grundlagenforschung als auch der angewandten Forschung zunimmt. All diese Technologien beruhen auf der synthetischen Generierung von RNA durch Amplifikation *in vitro*.

Die Herstellung von siRNA *in vitro* erfolgt bislang über chemische Synthese oder mit Hilfe von DNA-abhängigen RNA-Polymerasen wie der T7-RNA-Polymerase. Dafür werden zwei komplementäre DNA-Stränge mittels PCR unter Einsatz eines Primers, der auch die Sequenz für einen T7-Promotor enthält, synthetisiert. Nach Umschreiben der DNA mit Hilfe der T7 RNA-Polymerase werden die resultierenden komplementären RNA-Stränge hybridisiert und mittels RNAse I verdaut.

Im medizinischen Bereich spielt die RNA-Amplifikation eine wichtige Rolle beim Nachweis viraler Infektionen im Patientenmaterial. Weltweit werden 80% aller viralen Infektionen durch RNA-Viren, d.h. virale Erreger mit einem aus RNA bestehenden Erbgut, verursacht. Wichtige Erreger aus dieser Gruppe von Viren sind u.a. das Humane Immundefizienz-Virus (HIV), das Hepatitis-C-Virus (HCV), das Hepatitis-A-Virus, das Grippe-Virus (Influenza A, B und C) aber auch das Vogelpest-Virus, das vor kurzem neu Identifizierte SARS-Virus, das Virus der Kinderlähmung (Pollovirus), das Masern-Virus, das Mumps-Virus, das Röteln-Virus und die Brechdurchfall-Viren (Rotavirus, Sapovlrus und Norovirus).

Der Nachweis von RNA im Patientenmaterial findet bis dato in vier Schritten statt. Nach der Gewinnung der RNA aus dem Untersuchungsmaterial folgen die Schritte:
1. Umschreibung der RNA in copy-DNA (cDNA) durch RNA-abhänglge DNA-Polymerasen, die sog, reversen Transkriptasen. Dafür bedarf es auch des Einsatzes eines Primers. Primer sind Oligonukleotide, die spezifisch für die zu amplifizierende Sequenz sind. Primer und die RNA-abhängige DNA-Polymerase ermöglichen die gezielte Amplifikation vom Erbgut, allerdings in DNA-Form.
2. Amplifikation der cDNA durch Polymerasekettenreaktion (PCR),
3. Aufreinigung des PCR-Produkts,
4. Sequenzierung des PCR-Produkts.

Für die Herstellung von RNA, beispielsweise zum Expressionsnachweis oder zur Herstellung von siRNA, erfolgt zusätzlich die Umschreibung des PCR-Produkts in RNA durch DNA-abhängige RNA-Polymerasen, wie z.B. die T7-Polymerase.
Die Durchführung dieser Schritte dauert in der Regel 18 bis 24 Stunden. Ebenfalls dazu benötigt wird auch ein gewisses "Know-How", was nicht allen Forschungseinrichtungen zugänglich ist.

Eine weitere Methode zur Amplifikation von RNA ist die Replikation durch RNA-abhängige RNA-Polymerasen wie z.B. die RNA-Polymerase des Bakteriophagen Qβ (auch Qβ - Replikase genannt), die RNA-Polymerase des Poliovirus oder des Hepatitis-C-Virus.

Das Genom des Bakteriophagen Qβ besteht aus einer einzelsträngigen (+)-RNA, die durch die RNA-abhängige RNA-Polymerase repliziert wird. Die Qβ - Replikase stellt eine (-)-Strang-RNA-Kopie des (+)-Stranges her, weiche ebenso wie (+)-Stränge als Matrize fungieren kann. Auf diese Weise kann eine exponentielle Vervlelfältigung erreicht werden. Das Qβ -System wird bereits zum Nachweis von Analyten, wie etwa Nukleinsäuren, verwendet (Chu et al, (1986), Nucleic Acids Research 14, 5591 - 5603; Lizardi et al. (1988), Biotechnology 6, 1197 - 1202).

Diese RNA-abhängigen RNA-Polymerasen benötigen jedoch bestimmte RNA-Sequenzen zur Initiation der RNA-Replikation. Zudem sind sie für die Amplikation der RNA auch auf bestimmte Hilfsproteine angewiesen. Daher sind diese RNA-abhänglgen RNA-Polymerasen nicht dazu in der Lage, heterologe RNA zu amplifizieren.

Die WO 02/092774 A2 beschreibt ein Verfahren zur Amplifikation von RNA mit Hilfe eines Oligonukleotid-Primers, der eine so genannte SLC (stem-loop-cytosine)-Struktur aufweist. Der Erfindung liegt daher die Aufgabe zugrunde, ein Verfahren und Kits zur Amplifikation von viraler, eukaryontischer und prokaryontischer einzel- und doppelsträngiger Ribonukleinsäure (RNA) bereitzustellen, die effizienter und schneller sind.

Erfindungsgemäß wird die Aufgabe gelöst durch ein *In-vitro*-Verfahren zur Amplifikation von heterologer viraler, eukaryontischer oder prokaryontischer RNA gemäß Anspruch 1, wobei die RNA-abhängige RNA-Polymerase vorzugsweise eine RNA-abhängigie RNA-Polymerase eines Virus ist, der zu den Viren der Familie der *Caliciviridae* gehört, und eine "Recht-Hand-Konformation" aufweist und die Aminosäuresequenz der RNA-abhängigen RNA-Polymerase die folgenden Sequenzabschnitte umfasst:
a. XXDYS
b. GXPSG
c. YGDD
d. XXYGL
e. XXXXFLXRXX
mit der Bedeutung:
D: Aspartat
Y: Tyrosin
S: Serin
G: Glycin
P: Prolin
L: Leucin
F: Phenylalanin
R: Arginin
X: eine beliebige Aminosäure.

Unter einer sog. "Rechte-Hand-Konformation" ist dabei zu verstehen, dass die tertiäre Struktur (Konformation) des Proteins eine Faltung nach einer rechten Hand mit Finger (finger), Handfläche (palm) und Daumen (thumb), aufweist.

Bei dem Sequenzabschnitt "XXDYS" handelt es sich um das sog. A-Motiv. Das A-Motiv ist für die Diskriminierng zwischen Ribonukleosiden und Deoxyribonukleosiden zuständig.
Bei dem Sequenzabschnitt "GXPSG" handelt es sich um das sog. B-Motiv. Das B-Motiv ist in allen Vertretern der Familie *Caliciviridae* konserviert.
Bei dem Sequenzabschnitt "YGDD" handelt es sich um das sog. C-Motiv. Das C-Motiv stellt die aktive Stelle des Enzyms dar. Dieses Motiv spielt bei der Koordinierung der Metallionen eine wichtige Rolle. Bei dem Sequenzabschnitt "XXYGL" handelt es sich um das sog. D-Motiv. Das D- Motiv ist ein Merkmal der Template-abhänglgen Polymerasen.

Bei dem Sequenzabschnitt "XXXXFLXRXX" handelt es sich um das sog. E-Motiv. Das E-Motiv ist ein Merkmal der RNA-abhängigen RNA Polymerasen, und kommt bei DNA-abhängigen RNA-Polymerasen nicht vor.

Die erfindungsgemäß verwendete RNA-abhängige RNA-Polyemerase verfügt u.a. über die folgenden Funktionen:
- Mg²⁺- oder Mn²⁺-abhängige RNA-abhängige RNA-Polymeraseaktivität, keine DNA-Abhängigkeit
- Template-Abhängigkeit
- Primer-abhängige RNA-Synthese mit heteropolymerer RNA als Matrize.

Die Primer-abhängige Amplifikation der RNA erfolgt nur in der Anwesenheit eines Sequenzspezifischen Primers. Die Amplifikation ist Sequenz-abhängig und erfolgt durch Einbau von AMP, GMP, CMP oder UMP.

Überraschenderweise ist die erfindungsgemäß verwendete RNA-abhängige RNA-Polymerase in der Lage, *in vitro* heterologe virale, eukaryontische und prokaryontische heteropolymere RNA als Template für die Primer-unabhängige Amplifikationsreaktion zu nutzen. Sowohl positiv-strängige und negativ-strängige einzelsträngige als auch doppelsträngige RNA ist als Template zur Primer-unabhängigen Amplifikation einsetzbar.

Vorzugsweise ist die erfindungsgemäß verwendete RNA-abhängige RNA-Polymerase eine RNA-abhängige RNA-Polymerase eines Virus aus der Familie der *Caliciviridae.* Das Genom der *Caliciviridae* besteht aus einem polyadenylierten (+)strängigen RNA-Einzelstrang. *In vivo* schreibt die RNA-abhängige RNA-Polymerase des Calicivirus bei der viralen Replikation die genomische Calicivirus-RNA in eine (-)-strängige antisense-RNA (aRNA) um. Dabei entsteht ein RNA-aRNA-Hybrid. Die (-)-strängige aRNA dient im Anschluß als Templat zur Synthese neuer (+)-strängiger genomischer Virus-RNA.

In weiteren bevorzugten Ausführungsformen der Erfindung ist die RNA-abhängige RNA-Polymerase eine RNA-abhängige RNA-Polymerase eines humanen und / oder nicht-human pathogenen Calicivirus. Besonders bevorzugt handelt es sich um eine RNA-abhängige RNA-Polymerase eines Norovirus, Sapovirus, Vesivirus oder eines Lagovirus, z.B. die RNA-abhängige RNA-Polymerase des Norovirus-Stammes HuCV/NL/Dresden174/1997/GE oder des Sapovirus-Stammes pJG-Sap01 oder des Vesivirus-Stammes FCV/Dresden/2006/GE.

In besonders bevorzugten Ausführungsformen der Erfindung ist die RNA-abhängige RNA-Polymerase ein Protein mit einer Aminosäuresequenz gemäß **SEQ ID NO 1, SEQ ID NO 2** oder **SEQ ID NO 3.**
**SEQ ID NO 1** ist die Aminosäuresequenz einer Norovirus-RdRP.
**SEQ ID NO 2** ist die Aminosäuresequenz einer Sapovirus-RdRP.
**SEQ ID NO 3** ist die Aminosäuresequenz einer Vesivirus-RdRP.

SEQ ID NO 1:
MGGDSKGTYCGAPILGPGSAPKLSTKTKFWRSSTTPLPPGTYEPAYLGGK
DPRVKGGPSLQQVMRDQLKPFTEPRGKPPKPSVLEAAKKTIINVLEQTID
PPEKWSFTQACASLDKTTSSGHPHHMRKNDCWNGESFTGKLADQASKANL
MFEGGKNMTPVYTGALKDELVKTDKIYGKIKKRLLWGSDLATMIRCARAF
GGLMDELKAHCVTLPIRVGMNMNEDGPIIFERHSRYKYHYDADYSRWDST
QQRAVLAAALEIMVKFSSEPHLAQVVAEDLLSPSVVDVGDFKISINEGLP
SGVPCTSQWNSIAHWLLTLCALSEVTNLSPDIIQANSLFSFYGDDEIVST
DIKLDPEKLTAKLKEYGLKPTRPDKTEGPLVISEDLNGLTFLRRTVTRDP
AGWFGKLEQSSILRQMYWTRGPNHEDPSETMIPHSQRPIQLMSLLGEAAL
HGPAFYSKISKLVIAELKEGGMDFYVPRQEPMFRWMRFSDLSTWEGDRNL
APSFVNEDGVEVDKLAAALE

SEQ ID NO 2:
MKDEFQWKGLPVVKSGLDVGGMPTGTRYHRSPAWPEEQPGETHAPAPFGAGD
KRYTFSQTEMLVNGLKPYTEPTAGVPPQLLSRAVTHVRSYIETIIGTHRS
PVLTYHQACELLERTTSCGPFVQGLKGDYWDEEQQQYTGVLANHLEQAWD
KANKGIAPRNAYKLALKDELRPIEKNKAGKRRLLWGCDAATTLIATAAFK
AVATRLQVVTPMTPVAVGINMDSVQMQVMNDSLKGGVLYCLDYSKWDSTQ
NPAVTAASLAILERFAEPHPIVSCAIEALSSPAEGYVNDIKFVTRGGLPS
GMPFTSVVNSINHMIYVAAAILQAYESHNVPYTGNVFQVETVHTYGDDCM
YSVCPATASIFHAVLANLTSYGLKPTAADKSDAIKPTNTPVFLKRTFTQT
PHGVRALLDITSITRQFYWLKANRTSDPSSPPAFDRQARSAQLENALAYA
SQHGPVVFDTVRQIAIKTAQGEGLVLVNTNYDQALATYNAWFIGGTVPDP
VGHTEGTHKIVFEME

SEQ ID NO 3:
MKVTTQKYDVTKPDISYKGLICKQLDEIRVIPKGTRLHVSPAHTDDYDEC
SHQPASLGSGDPRCPKSLTAIVVDSLKPYCEKTDGPPHDILHRVQRMLID
HLSGFVPMNISSEPSMLAAFHKLNHDTSCGPYLGGRKKDHMIGGEPDKPL
LDLLSSKWKLATQGIGLPHEYTIGLKDELRPVEKVQEGKRRMIWGCDVGV
ATVCAAAFKGVSDAITANHQYGPVQVGINMDGPSVEALYQRIRSAAKVFA
VDYSKWDSTQSPRVSAASIDILRYFSDRSPIVDSAANTLKSPPIAIFNGV
AVKVTSGLPSGMPLTSVINSLNHCLYVGCAILQSLESRNIPVTWNLFSTF
DMMTYGDDGVYMFPMMFASVSDQIFANLTAYGLKPTRVDKSVGAIEPIDP
ESVVFLKRTITRTPHGIRGLLDRGSIIRQFYYIKGENSDDWKTPPKTIDP
TSRGQQLWNACLYASQHGPEFYNKVYRLAEKAVEYEELHFEPPSYHSALE
HYNNQFNGVDTRSDQIDASVMTDLHCDVFEVLE

Die RNA-abhängigen RNA-Polymerasen des Norovirus, des Sapovirus und des Vesivivirus sind rekombinant herstellbar durch Klonierung der die Norovirus-, Sapovirus- bzw. Vesivirus-RdRP kodierenden Nukleinsäure in einen geeigneten Expressionsvektor, beispielsweise pET-28 (Novagen). Der Expressionsvektor mit der Gensequenz, die für die Virus-RdRP kodiert, wird zur Expression in einen geeigneten Wirtsorganismus eingebracht. Der Wirtsorganismus wird bevorzugt aus den üblicherweise für die Proteinexpression verwendeten prokaryontischen, bevorzugt *Eschericha coli*, oder eukaryontischen Wirtsorganismen, bevorzugt *Sacharomyces cerevisae* oder Insektenzellen (bevorzugt Sf9-Zellen), die mit rekombinanten Baculoviren infiziert worden sind, gewählt. Dieser Wirtsorganismus enthält mindestens einen Expressionsvektor mit einer Gensequenz, die für die Virus-RdRP kodiert.

In bevorzugten Ausführungsformen wird die Norovirus-, Sapovirus- oder Vesivirus- Calicivirus-RdRP am N- oder C-Terminus mit einer Proteinsequenz fusioniert, welche die Aufreinigung des RdRP-Fusionsproteins nach der Expression in einem Wirtsorganismus erleichtert. Bevorzugt ist diese Sequenz ein sogenannter Histidin-Marker, bestehend aus einer Sequenz von mindestens 6 aufeinanderfolgenden Histidinen (His oder H). Ein solcher Histidin-Marker ermöglicht in bekannter Weise die Aufreinigung des Proteins affinitätschromatographisch auf einer Nickel- oder Kobalt-Säule.

Beispiele für mit einem Histidin-Marker fusionierte Ausführungsformen der RNA-abhänglgen RNA-Polymerase sind die Proteine mit einer Aminosäuresequenz gemäß **SEQ ID NO 4, SEQ 10 NO 5** oder **SEQ ID NO 6.**

**SEQ ID NO 4** ist die Aminosäuresequenz einer Norovirus-RdRP mit einem Histidin-Marker (His-tag). **SEQ ID NO 5** ist die Aminosäuresequenz einer Sapovirus-RdRP mit einem Histidin-Marker (His-tag). **SEQ ID NO 6** ist die Aminosäuresequenz einer Vesivirus-RdRP mit einem Histidin-Marker (His-tag).

SEQ ID NO 4:
MGGDSKGTYCGAPILGPGSAPKLSTKTKFWRSSTTPLPPGTYEPAYLGGK

DPRVKGGPSLQQVMRDQLKPFTEPRGKPPKPSVLEAAKKTIINVLEQTID
PPEKWSFTQACASLDKTTSSGHPHHMRKNDCWNGESFTGKLADQASKANL
MFEGGKNMTPVYTGALKDELVKTDKIYGKIKKRLLWGSDLATMIRCARAF
GGLMDELKAHCVTLPIRVGMNMNEDGPIIFERHSRYKYHYDADYSRWDST
QQRAVLAAALEIMVKFSSEPHLAQVVAEDLLSPSVVDVGDFKISINEGLP
SGVPCTSQWNSIAHWLLTLCALSEVTNLSPDIIQANSLFSFYGDDEIVST
DIKLDPEKLTAKLKEYGLKPTRPDKTEGPLVISEDLNGLTFLRRTVTRDP
AGWFGKLEQSSILRQMYWTRGPNHEDPSETMIPHSQRPIQLMSLLGEAAL
HGPAFYSKISKLVIAELKEGGMDFYVPRQEPMFRWMRFSDLSTWEGDRNL
APSFVNEDGVEVDKLAAALEHHHHHH

SEQ ID NO 5:
MKDEFQWKGLPVVKSGLDVGGMPTGTRYHRSPAWPEEQPGETHAPAPFGAGD
KRYTFSQTEMLVNGLKPYTEPTAGVPPQLLSRAVTHVRSYIETIIGTHRS
PVLTYHQACELLERTTSCGPFVQGLKGDYWDEEQQQYTGVLANHLEQAWD
KANKGIAPRNAYKLALKDELRPIEKNKAGKRRLLWGCDAATTLIATAAFK
AVATRLQVVTPMTPVAVGINMDSVQMQVMNDSLKGGVLYCLDYSKWDSTQ
NPAVTAASLAILERFAEPHPIVSCAIEALSSPAEGYVNDIKFVTRGGLPS
GMPFTSVVNSINHMIYVAAAILQAYESHNVPYTGNVFQVETVHTYGDDCM
YSVCPATASIFHAVLANLTSYGLKPTAADKSDAIKPTNTPVFLKRTFTQT
PHGVRALLDITSITRQFYWLKANRTSDPSSPPAFDRQARSAQLENALAYA
SQHGPVVFDTVRQIAIKTAQGEGLVLVNTNYDQALATYNAWFIGGTVPDP
VGHTEGTHKIVFEMEHHHHHH

SEQ ID NO 6:
MKVTTQKYDVTKPDISYKGLICKQLDEIRVIPKGTRLHVSPAHTDDYDEC
SHQPASLGSGDPRCPKSLTAIVVDSLKPYCEKTDGPPHDILHRVQRMLID
HLSGFVPMNISSEPSMLAAFHKLNHDTSCGPYLGGRKKDHMIGGEPDKPL
LDLLSSKWKLATQGIGLPHEYTIGLKDELRPVEKVQEGKRRMIWGCDVGV
ATVCAAAFKGVSDAITANHQYGPVQVGINMDGPSVEALYQRIRSAAKVFA
VDYSKWDSTQSPRVSAASIDILRYFSDRSPIVDSAANTLKSPPIAIFNGV
AVKVTSGLPSGMPLTSVINSLNHCLYVGCAILQSLESRNIPVTWNLFSTF
DMMTYGDDGVYMFPMMFASVSDQIFANLTAYGLKPTRVDKSVGAIEPIDP
ESVVFLKRTITRTPHGIRGLLDRGSIIRQFYYIKGENSDDWKTPPKTIDP
TSRGQQLWNACLYASQHGPEFYNKVYRLAEKAVEYEELHFEPPSYHSALE
HYNNQFNGVDTRSDQIDASVMTDLHCDVFEVLEHHHHHH

Die vorliegende Erfindung bezieht sich auf ein *In-vitro*-Verfahren zur primer-abhängigen Amplifikation von heterologer viraler, eukaryontischer oder prokaryontischer RNA mittels einer RNA-abhängigen RNA-Polyermerase gemäß der Definition im Anspruch 1 mit den Schritten
a. Anlagerung der RNA-abhängigen RNA-Polymerase an die RNA-Matrize in der Anwesenheit eines sequenzspezifischen Oligonukleotid-Primers, z.B. eines Oligo-RNA- oder Oligo-DNA-Primersder mit einem Abschnitt der RNA-Matrize hybridisiert,
b. Umschreiben der RNA-Matrize in antisense-RNA, wobei der Primer durch die RNA-abhängige RNA-Polymerase entsprechend der Sequenz der RNA-Matrize verlängert wird,
c. Trennung des RNA/antisense-RNA-Duplexes in einzelne Stränge durch Hitzedenaturierung, chemische Denaturierung und/oder ein Enzym mit der Fähigkeit, doppelsträngige RNA in einzelsträngige RNA aufzutrennen
sowie ein Kit zur Durchführung des Verfahrens zur primer-abhängigen Amplifikation von heterologer viraler, eukaryontischer oder prokaryontischer RNA gemäß Anspruch 13.

Die RNA-Matrize wird beim erfindungsgemäßen Verfahren vorzugsweise in Mengen von 1 µg bis 4 µg pro 50 µl-Reaktionsansatz eingesetzt. Die Konzentration der eingesetzten Ribonukleotide ATP, CTP, GTP und UTP (NTPs) beträgt vorzugsweise zwischen 0,1 µmol/l und 1 µmol/l, besonders bevorzugt 0,4 µmol/l. Auch NTP-Analoga sind beim erfindungsgemäßen Verfahren zur RNA-Amplifikation einsetzbar, beispielsweise fluoreszenzmarklerte NTPs, Biotin-markierte NTPs oder radioaktiv markierte NTPs wie [P³²]-markierte NTPs. Die Konzentration der erfindungsgemäß verwendeten RdRP beträgt bevorzugt zwischen 1 µmol/l und 3µmol/l.

Das Verfahren wird vorzugsweise bei einem pH-Wert zwischen 6,5 und 8,5 und bei einer Temperatur zwischen 20°C und 40°C durchgeführt. Eine bevorzugte Ausführungsform des Verfahrens zur RNA-Amplifikation wird bei 30°C mit Norovirus-RdRP oder bei 37°C mit Sapovirus-RdRP unter folgenden Pufferbedingungen durchgeführt: 50 mmol/l HEPES, pH 8.0, 3 mmol/l Magnesiumacetat oder Manganchlorid, 4 mmol/l DTT.

Erfindungsgemäß erfolgt die Trennung des RNA / antisense-RNA-Duplexes in einzelne RNA-Stränge durch Hitzedenaturierung, chemische Denaturierung und/oder mit einem Enzym mit der Fähigkeit, doppelsträngige RNA in einzelsträngige RNA aufzutrennen wie z.B. eine Helikase.

Durch die Strangtrennung liegt wieder einzelsträngiges Template vor und eine weitere Amplifikationsrunde kann eingeleitet werden.
Durch den Einsatz eines Enzyms mit der Fähigkeit, doppelsträngige RNA in einzelsträngige RNA aufzutrennen, kann die Reaktion isotherm gehalten werden.

Bestandteil der Erfindung ist ein entsprechendes Kit zur primer-abhängigen Amplifikation von heterologer viraler, eukaryontischer oder prokaryontischer RNA, das ein Enzym mit der Fähigkeit, doppelsträngige RNA in einzelsträngige RNA aufzutrennen wie z.B. eine Helikase, enthält.

Die Erfindung betrifft ein Verfahren gemäß Anspruch 1 zur primer-abhängigen Amplifikation von heterologer viraler, eukaryontischer oder prokaryontischer RNA, wobei mindestens ein Primer, der mit einem Abschnitt der RNA-Matrize hybridisiert, eingesetzt und durch die RNA-abhängige RNA-Polymerase entsprechend der Sequenz der RNA-Matrize verlängert wird.

Als Primer wird bevorzugt ein RNA-Primer oder ein DNA-Primer, z.B. mit einer Länge von 20 bis 25 Basen, vorzugsweise in einer Konzentration von 0,1 bis 1 µmol/l eingesetzt. Als RNA-Matrize ist dabei z.B. virale, prokaryontische und eukaryontische RNA einsetzbar.

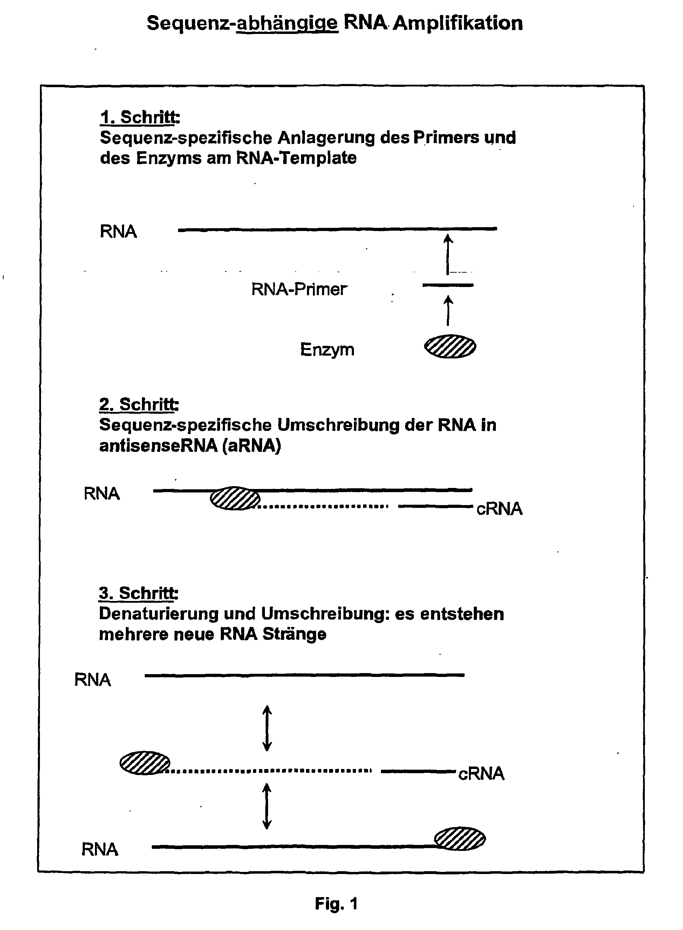

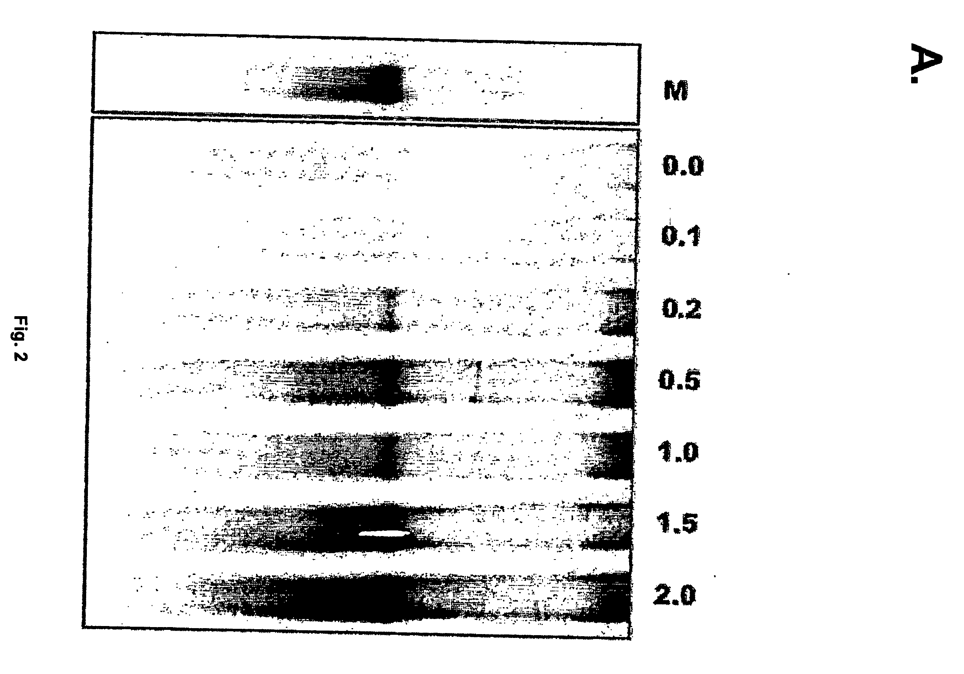

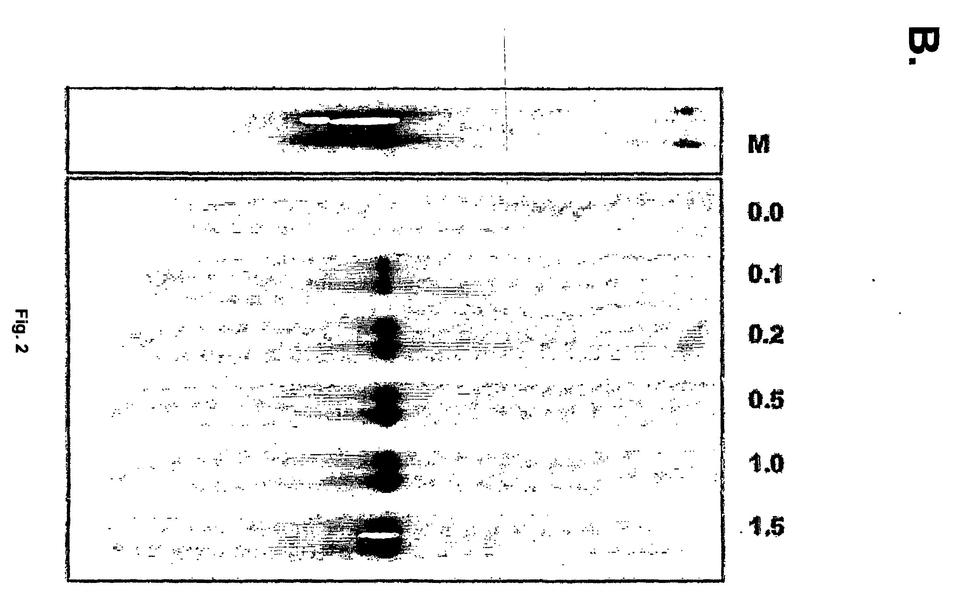

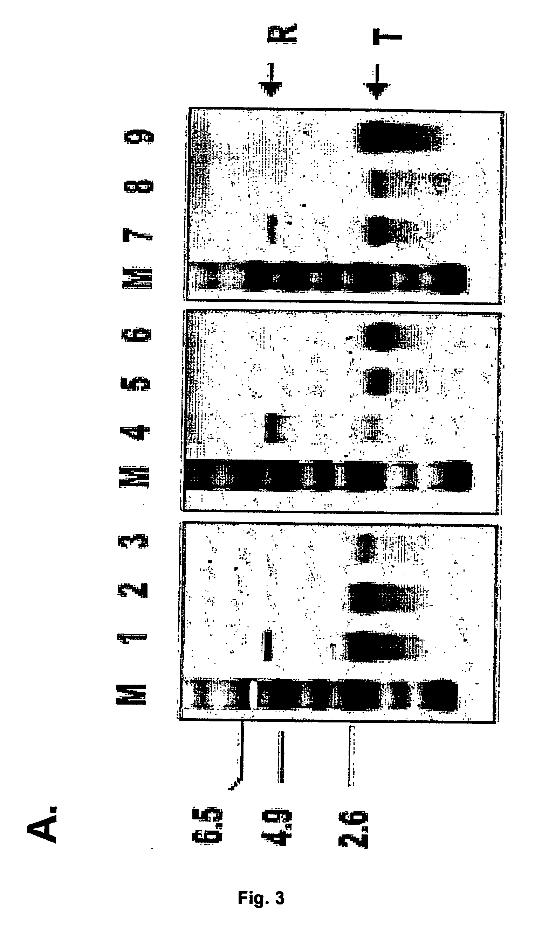

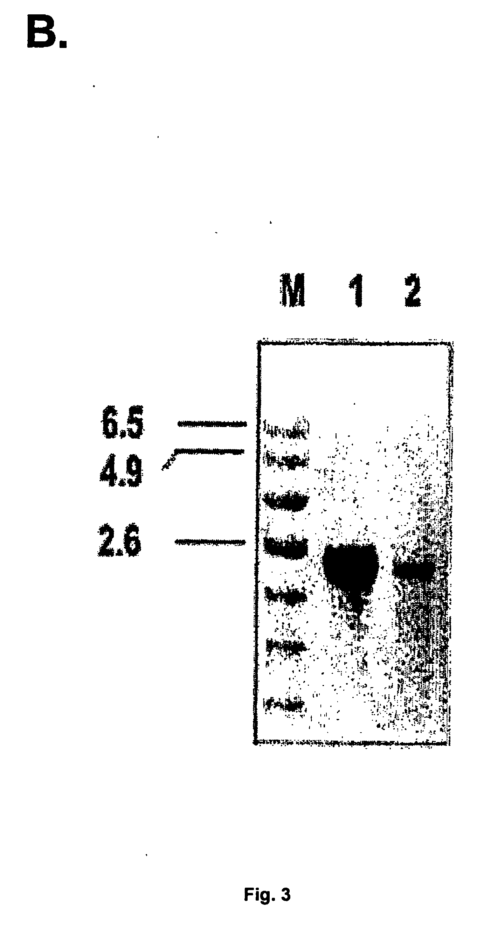

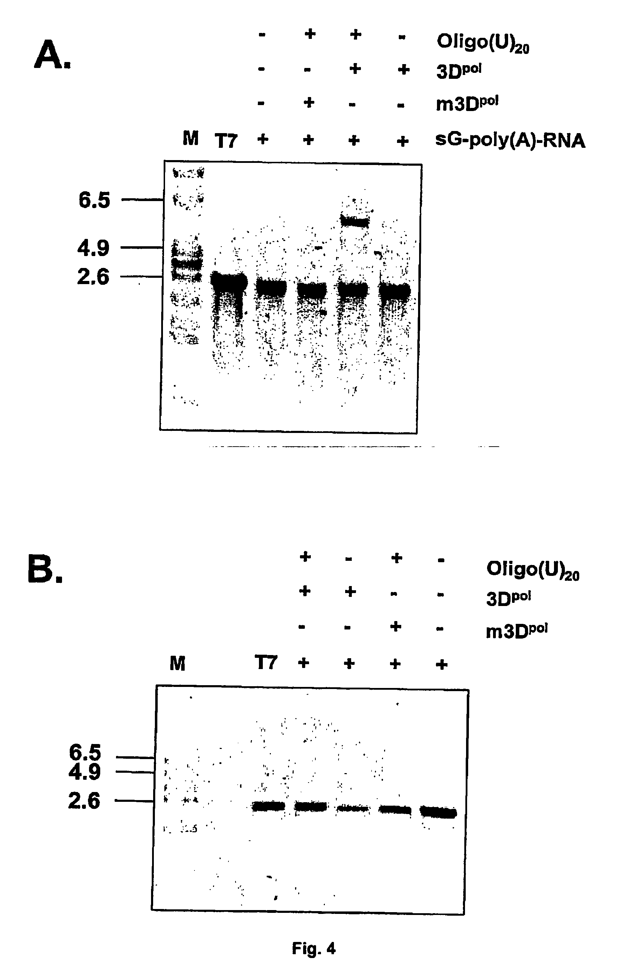

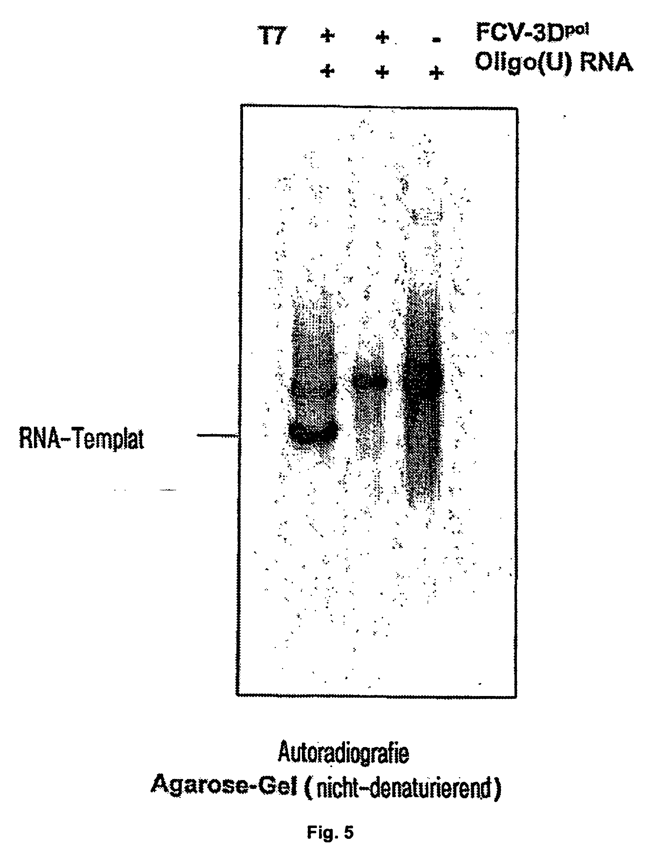

Der Ablauf der primerabhängigen RNA-Amplifikation einer einzelsträngigen RNA-Matrize unter Einsatz eines Primers ist in **Figur 1** schematisch dargestellt.

Vorzugsweise wird bei polyadenylylierter RNA, polyurydylilierter RNA, polyguanylylierter RNA oder polycytidylyllerter RNA als Primer ein poly-U-RNA, poly-A-RNA, poly-C-RNA oder poly-G-RNA-Primer eingesetzt. Mit Hilfe des poly-U-RNA-Primers ist eine spezifische Amplifikation der gesamten zellulären mRNA möglich. Vorzugsweise wird dafür ein Poly-U-Primer von 20 bis 24 Basen Länge eingesetzt. Die amplifizierte zelluläre mRNA kann Anschluss beispielsweise mit Hilfe der sog. Microarray-Verfahren untersucht werden.

Das erfindungsgemäße Verfahren zur sequenzspezifischen RNA-Amplifikation wird vorteilhaft auch zum Nachweis viraler RNA in Patientenmaterial verwendet.

Hierfür wird die gesamte zelluläre RNA aus dem Patientenmaterial gewonnen und unter Einsatz eines RNA-Primers, der spezifisch mit einem bestimmten Abschnitt viraler RNA hybridisiert, wird im Patientenmaterial vorhandene virale RNA amplifiziert.

Als Patientenmaterial wird beispielsweise Liquor, Blut, Plasma oder Körperflüssigkeiten eingesetzt.

Auch geeignet ist das Verfahren zum Nachweis von RNA-Viren mit einem poly-A-Schwanz am 3'-Ende des Genoms, DNA-Viren und viralen mRNA-Transkripten.

Bestandteil der Erfindung ist ebenfalls ein Kit zur Durchführung des Verfahrens zur primer-abhängigen Amplifikation von heterologer viraler, eukaryontischer oder prokaryontischer RNA, mindestens bestehend aus
a. einer RNA-abhängigen RNA-Polyermase gemäß der Definition in Anspruch 1
b. einem geeigneten Reaktions-Puffer
c. NTPs
d. einem Enzym mit der Fähigkeit, doppelsträngige RNA in einzelsträngige RNA aufzutrennen
e. Oligonukleotid-Primer
f. ggf. RNase-Inhibitor
g. ggf. Stoplösung.
In einer besonders bevorzugten Ausführungsform enthält das Kit
a. 150 rekombinant hergestellter µmo/l Norovirus-RdRP gemäß SEQ **ID** NO 1 oder **SEQ ID** NO 4 und/oder Sapovirus-RdRP gemäß **SEQ ID NO 2** oder **SEQ ID NO 5** und/oder Vesivirus-RdRP gemäß **SEQ ID NO 3** oder **SEQ ID NO 6**
b. als Puffer: 50 mmol/l HEPES, pH 8.0, 3 mmol/l Magnesiumacetat oder Manganchlorid (MnCl₂), 4 mM DTT
c. 10 mmol/l ATP, 10 mmol/l CTP, 10 mmol/l GTP,10 mmol/l UTP
d. RNase-Inhibitor
e. als Stoplösung: 4 mol/l Ammoniumacetat, 100 mmol/l EDTA
f. 0,1 bis 3 µM Primer (homopolymeres oder heteropolymeres RNA- oder DNA-Oligonukleotid mit einer Länge von 15 bis 20 Basen)
g. ein Enzym mit der Fähigkeit, doppelsträngige RNA in einzelsträngige RNA aufzutrennen.

Die Anwendungsmöglichkeiten der erfindungsgemäßen Verfahren zur Amplifkation von RNA sind vielfältig. Zum einem ist der direkte Nachweis viralen Erbgutes in Patientenmaterial möglich. Hierfür wird die gesamte zelluläre RNA gewonnen und unter Einsatz eines RNA-Primers eine beliebige RNA-Sequenz spezifisch amplifiziert. Der Nachweis der viralen Nukleinsäure erfolgt dann durch Hybridisierung an spezifischen Sonden. Eine andere Anwendung betrifft die unspezifische (RNA-Primer-unabhängige) Amplifikation der RNA, die ermöglichen kann, bisher nicht identifizierte Viren bzw. neue virale Varianten zu identifizieren.

Eine weitere Anwendung betrifft die Microarray-Technologie. Diese hat den differenziellen Nachweis der zellulären Expression zum Ziel. Dies geschieht durch den Nachweis der sog. zellulärer Transkripte, d.h. der mRNA. Die Erfindung ermöglicht ausgehend aus einem zellulären Gemisch, die mRNA spezifisch durch den Einsatz eines poly(U)-Oligonukleotid zu amplifizieren, um anschließend auf Microarrays durch Hybridisierung den Nachweis zu erbringen.

Anhand der folgenden Figuren und Ausführungsbeispiele wird die Erfindung näher erläutert. Dabei zeigen
**Figur 1****:**
   **Schematischer Ablauf der sequenz-abhängigen RNA-Amplifikation**
**Figur 2****:**
   **Primer-abhängige Replikation von *full-length* subgenomischer polyadenylierter RNA durch Norovirus 3D**^{**pol**.} Bei allen Reaktionen wurde synthetische subgenomische polyadenylierte RNA als Template eingesetzt. Die Reaktionsprodukte wurden auf Formaldehyd-Agarosegelen analysiert und durch Autoradiographie visualisiert.
   **(A)** Die Reaktion wurde in Gegenwart eines oligo(U)₂₀-RNA-Primers wie angegeben (mM) durchgeführt. M, Marker (*in vitro* transkribierte subgenomische polyadenylierte RNA).
   **(B)** Die Reaktion wurde in Gegenwart eines RNA-Oligonukleotids durchgeführt, das komplementär zu der dem poly(A)-Schwanz benachbarten Sequenz ist. RNA-Oligonukleotid Primerkonzentration (mM) wie angegeben. M, Marker (*in* vitro transkribierte subgenomische polyadenylierte RNA).
**Figur 3****:**
   **RNA-Synthese an Norovirus anti-subgenomischer RNA.**
   Bei allen Reaktionen wurde synthetische anti-subgenomische RNA als Template für die Replikationsreakton eingesetzt. Die RNA-Reaktionsprodukte wurden auf nativen Agarosegelen analysiert und nach Ethidiumbromidfärbung durch UV-Transillumination visualisiert.
   **(A)** Bahnen 4 bis 6: RNA-Synthese in Gegenwart von Wildtyp 3D^{pol} und einem RNA-Oligonukleotid komplementär zum 3'-Terminus, mutierter 3D^{pol} und einem RNA-Oligonukleotid komplementär zum 3'-Terminus bzw. mit einem RNA-Oligonukleotid komplementär zum 3'-Terminus, aber ohne Norovirus 3D^{pol}. Bahnen 7 bis 9, RNA-Synthese in Gegenwart von Wildtyp 3D^{pol} und einem DNA-Oligonukleotid komplementär zum 3'-Terminus, mutierter 3D^{pol} und einem DNA-Oligonukleotid komplementär zum 3'-Terminus, bzw. mit einem DNA-Oligonukleotid komplementär zum 3'-Terminus, aber ohne Norovirus 3D^{pol}. T, Template-RNA. R, Replikationsprodukt. M, RNA-Molekulargewichtsmarker (Kb).
   **(B)** Strangseparationsanalyse der Replikationsprodukte. Die Reaktionsprodukte wurden auf Formaldehyd-Agarosegelen analysiert und nach Ethidiumbromidfärbung durch UV-Transillumination visualisiert. Bahnen 1 und 2, Template (anti-subgenomische RNA) bzw. Norovirus 3D^{pol} Replikationsprodukt. M, RNA Molekulargewichtsmarker (Kb).
**Figur 4****:**
   **RNA-Synthese mit Sapovirus 3D^{pol}.**
   **(A)** Die RNA-Synthese in Gegenwart einer synthetischen subgenomischen polyadenylierten RNA (sG-poly(A)-RNA) als Template wurde mit und ohne Zugabe eines 3 µM oligo(U)₂₀ RNA-Primers und in Anwesenheit von Wildtyp Sapovirus 3D^{pol} (3D^{pol}) oder wie angegeben einer in der 'acitve site' mutierten 3D^{pol} (m3D^{pol}, YGD_{343G}D_{344G}) untersucht. Die Reaktionsprodukte wurden auf nicht-denaturierenden Agarosegelen analysiert und nach Ethidiumbromidfärbung mit UV-Transillumination visualisiert. Die sichtbare verbleibende Bande in den Reaktionen, bei denen m3D^{pol} anstelle von Wildtyp 3D^{pol} eingesetzt wurden oder bei denen kein Oligo(U)₂₀ RNA-Primer zugegben wurde, resultiert vom in der Reaktion eingesetzten synthetischen subgenomischen RNA Template. T7, synthetische subgenomische RNA, die mittels *in vitro* Transkription mit T7 hergestellt wurde. M, RNA-Molekulargewichtsmarker.
   **(B)** Strangseparationsanalyse des Reaktionsprodukts der in vitro RNA-Synthese mit Sapovirus 3D^{pol}. Das Reaktionsprodukt wurde wie angegeben durch RNA-Synthese mit Sapovirus 3D^{pol} (3D^{pol}) unter Verwendung einer subgenomeischen polyadenylierten RNA (sG-Poly(A)-RNA) als Template erhalten. Die Reaktionsprodukte wurden auf denaturierenden Formaldehyd-Agarosegelen visualisiert. T7, synthetische subgenomische RNA, die mittels *in vitro* Transkription mit T7 hergestellt wurde. M, RNA-Molekulargewichtsmarker.
**Figur 5****:**
   **RNA-Synthese mit Vesivirus 3D^{pol}.**
   **(A)** Die RNA-Synthese in Gegenwart einer synthetischen subgenomischen polyadenylierten RNA (sG-poly(A)-RNA) als Template wurde mit und ohne Zugabe eines 3 µM oligo(U)₂₀ RNA-Primers und in Anwesenheit von Wildtyp Vesivirus 3D^{pol} (3D^{pol}) untersucht. Die Reaktionsprodukte wurden auf nicht-denaturierenden Agarosegelen analysiert und nach Autoradiographie visualisiert. T7, synthetische subgenomische RNA, die mittels *in vitro* Transkription mit T7 hergestellt wurde. M, RNA-Molekulargewichtsmarker.

### Ausführungsbeispiel 1:

### Verfahren zur Herstellung rekombinanter Norovirus RdRP

Die cDNA der Norovirus-RdRP wurde durch PCR aus dem Norovirus-Klon pUS-Noril (Genbank accession number: AY741811) erhalten. Sie wurde in den pET-28b(+)-Vektor (Novagen) kloniert, der Expressionsvektor wurde sequenziert und in E.coli CL21(DE3)pLysS transformiert. Die Zellen wurden bei 37°C in Luria-Bertani-Medium mit Kanamycin (50 mg/L) kultiviert. Bei einer optischen Dichte von 0.6 (OD600) wurde die Proteinexpression durch den Zusatz von Isopropyl-β-D-thiogalactopyranosid (IPTG) mit einer Endkonzentration von 1 mM induziert. Die Kulturen wurden dann bei 25 °C über Nacht inkubiert. Die aus 250 ml Kultur erhaltenen Zellpellets wurden einmal in 4 ml phosphate buffered saline (PBS) und 1 % Triton X 100 (Sigma) gewaschen. Die Zellen wurden bei 37 °C für 15 Minuten mit DNase (10 U/ml) behandelt, auf Eis sonifiziert und in 40 ml Bindungspuffer (20 mM Tris/HCl, pH 7.9, 500 mM NaCl, 5 mM Imidazol) resuspendiert. Nach der Zentrifugation bei 4300 rpm bei 4 °C für 40 Minuten wurde das geklärte Lysat erhalten. Die mit einem His6-tag versehene Norovirus-RdRP wurde an eine Ni-Nitrilotriacetessigsäure (NTA)-Sepharosematrix (Novagen) gebunden, die mit dem Bindungspuffer prääquilibriert worden war. Das gebundene Protein wurde mit dem Bindungspuffer, der 60 mM Imidazol enthielt, gewaschen und mit Bindungspuffer, der 1 M Imidazol enthielt, eluiert. Das eluierte Protein wurde gegen Puffer A dialysiert (25 mM Tris-hCl, pH 8.0, 1 mM β-Mercaptoethanol, 100 mM NaCl, 5 mM MgCl2, 10 % Glycerin, 0.1 % Triton X). Die Proteinkonzentration wurde mit dem BCA proteinassay Kit (Pierce) auf der Grundlage der Biuret-Reaktion bestimmt. Das Enzym wurde in einem Endvolumen von 50 % Glycerin resuspendiert und bei -20°C aufbewahrt.

### Ausführungsbeispiel 2:

### Sequenzabhängige RNA-Amplifikation mit Norovirus-RdRP mit einem genspezifischen RNA-Primer

Die Reaktionsmischung (50 µl) besteht aus 0.5 bis 1 µg RNA-Matrize, 10 µl des Reaktionspuffer (250 mM HEPES, pH 8.0, 15 mM Magnesiumacetat, 20 mM DTT), 50 U RNase-inhibitor (RNAsin, Promega), je 0.4 mM von ATP, CTP, GTP, UTP, 0.1 bis 1 µM genspezifischer RNA-Primer, 3 µM der nach Ausführungsbeispiel 1 hergestellten Norovirus-RdRP. Die Reaktion wird bei 30 °C für 2 Stunden durchgeführt. Die Reaktion wird durch die Zugabe von 50 µl der Stoplösung (4 M Ammoniumacetat, 100 mM EDTA) gestoppt. Die Reinigung erfolgt durch Phenol-Chloroform-Extraktion oder mit dem MEGAclear Kit (Ambion) nach den Herstelleranweisungen. Die Transkriptionsprodukte werden nach Ethidiumbromid-Färbung durch UV-Transillumination auf TBE-gepufferten Agarosegelen sichtbar gemacht. Formamid-Agarosegele oder Harnstoff/Polyacrylamidgele können ebenfalls verwendet werden.

### Ausführungsbeispiel 3:

### Amplifikation der zellulären mRNA mit Norovirus-RdRP mit einem poly-U-Primer

Die Reaktionsmischung (50 µl) besteht aus 0.5 bis 1 µg RNA-Matrize, 10 µl des Reaktionspuffer (250 mM HEPES, pH 8.0, 15 mM Magnesiumacetat, 20 mM DTT), 50 U RNase-Inhibitor (RNAsin, Promega), je 0.4 mM von ATP, CTP, GTP, UTP, 0.1 bis 1 µM poly-(U)₂₀-Primer, 3 µM der nach Ausführungsbeispiel 1 hergestellten Norovirus-RdRP. Die Reaktion wird bei 30 °C für 2 Stunden durchgeführt. Die Reaktion wird durch die Zugabe von 50 µl der Stoplösung (4 M Ammoniumacetat, 100 mM EDTA) gestoppt. Die Reinigung erfolgt durch Phenol-Chloroform-Extraktion oder mit dem MEGAclear Kit (Ambion) nach den Herstelleranweisungen. Die Transkriptionsprodukte werden nach Ethidiumbromid-Färbung durch UV-Transillumination auf TBE-gepufferten Agarosegelen sichtbar gemacht. Formamid-Agarosegele können ebenfalls verwendet werden.

## Patentansprüche

1. Verfahren zur *In-vitro*-Amplifikation von heterologer viraler, eukaryontischer oder prokaryontischer RNA mit den Schritten
a. Anlagerung einer RNA-abhängigen RNA-Polymerase an die RNA-Matrize in Anwesenheit eines sequenzspezifischen Oligonukleotid-Primers, der mit einem Abschnitt der RNA-Matrize hybridisiert,
b. Umschreiben der RNA-Matrize in antisense-RNA, wobei der Primer durch die RNA-abhängige RNA-Polymerase entsprechend der Sequenz der RNA-Matrize verlängert wird,
c. Trennung des RNA/antisense-RNA-Duplexes in einzelne RNA-Stränge durch Hitzedenaturierung, chemische Denaturierung und/oder ein Enzym mit der Fähigkeit, doppelsträngige RNA in einzelsträngige RNA aufzutrennen,
d. ggf. Wiederholung der Schritte a. bis c.,
wobei die RNA-abhängige RNA-Polymerase eine "Rechte-Hand-Konformation" aufweist und die Aminosäuresequenz der RNA-abhängigen RNA-Polymerase die folgenden Sequenzabschnitte umfasst:
a. XXDYS
b. GXPSG
c. YGDD
d. XXYGL
e. XXXXFLXRXX
mit der Bedeutung:
D: Aspartat
Y: Tyrosin
S: Serin
G: Glycin
P: Prolin
L: Leucin
F: Phenylalanin
R: Arginin
X: eine beliebige Aminosäure.

2. Verfahren nach Anspruch 1, wobei die RNA-abhängige RNA-Polymerase eine RNA-abhängige RNA-Polymerase eines Virus aus der Familie der *Caliciviridae* ist.

3. Verfahren nach Anspruch 2, wobei die RNA-abhängige RNA-Polymerase eine RNA-abhängige RNA-Polymerase eines Norovirus, Sapovirus, Vesivirus oder Lagovirus ist.

4. Verfahren nach Anspruch 3, wobei die RNA-abhängige RNA-Polymerase ein Protein gemäß SEQ ID NO: 1, SEQ ID NO: 2, SEQ ID NO: 3, SEQ ID NO: 4, SEQ ID NO: 5 oder SEQ ID NO: 6 ist.

5. Verfahren nach einem der Ansprüche 1 bis 4, bei dem NTP-Analoga eingesetzt werden.

6. Verfahren nach einem der Ansprüche 1 bis 5, wobei die enzymatische Trennung des RNA/antisense-RNA-Duplexes durch eine Helikase erfolgt.

7. Verfahren nach einem der Ansprüche 1 bis 6, wobei der Primer ein heteropolymerer oder homopolymerer RNA- oder DNA-Primer ist.

8. Verfahren nach Anspruch 7, wobei der Primer eine Länge von 20 bis 25 Basen aufweist.

9. Verfahren nach Anspruch 7 oder 8, wobei der homopolymere Primer ein poly-U-RNA-Primer und die RNA-Matrize zelluläre Gesamt-RNA ist, so dass eine spezifische Amplifikation der gesamten zellulären mRNA erfolgt.

10. Verfahren nach Anspruch 7 oder 8, wobei die RNA-Matrize zelluläre Gesamt-RNA ist und der heteropolymere Primer spezifisch mit einem bestimmten Abschnitt viraler RNA hybridisiert.

11. Verfahren nach Anspruch 10, wobei vor dem Schritt a. die zelluläre Gesamt-RNA aus einem Patientenmaterial gewonnen wird.

12. Verfahren nach Anspruch 11, wobei das Patientenmaterial aus der Gruppe, bestehend aus Liquor, Blut, Plasma oder Körperflüssigkeiten, ausgewählt ist.

13. Kit zur Primer-abhängigen Amplifikation von heterologer viraler, eukaryontischer oder prokaryontischer RNA, mindestens bestehend aus
a. einer RNA-abhängigen RNA-Polymerase gemäß der Definition in einem der Ansprüche 1 bis 4,
b. einem geeigneten Reaktionspuffer,
c. NTPs,
d. einem Enzym mit der Fähigkeit, doppelsträngige in einzelsträngige RNA aufzutrennen, vorzugsweise einer Helikase,
e. Oligonnukleotid-Primer,
f. ggf. RNAse-Inhibitor,
g. ggf. Stopp-Lösung.

## Claims

1. A method for the amplification of heterologous viral, eukaryotic or prokaryotic RNA comprising the steps of:
a. annealing of an RNA-dependent RNA polymerase to the RNA template in the presence of a sequence-specific oligonucleotide primer hybridising with a segment of the RNA template;
b. transcribing the RNA template into antisense RNA wherein the primer is elongated by the RNA-dependent RNA polymerase according to the sequence of the RNA template;
c. separating the RNA/antisense RNA duplex into individual RNA strands by heat denaturation, chemical denaturation and/or by an enzyme capable of separating double-stranded RNA into single-stranded RNA;
d. optionally, repeating steps a. to c;
wherein the RNA-dependent RNA polymerase has a "right hand conformation" and wherein the amino acid sequence of the RNA-dependent RNA polymerase comprises the following sequence motifs:
a. XXDYS
b. GXPSG
c. YGDD
d. XXYGL
e. XXXXFLXRXX
with the following meanings:
D: aspartate
Y: tyrosine
S: serine
G: glycine
P: proline
L: leucine
F: phenylalanine
R: arginine
X: any amino acid.

2. The method of claim 1 wherein the RNA-dependent RNA polymerase is an RNA-dependent RNA polymerase of a virus of the *Caliciviridae* family.

3. The method of claim 2 wherein the RNA-dependent RNA polymerase is an RNA-dependent RNA polymerase of a norovirus, sapovirus, vesivirus or lagovirus.

4. The method of claim 3 wherein the RNA-dependent RNA polymerase is a protein according to SEQ ID NO:1, SEQ ID NO: 2, SEO ID NO: 3, SEO ID NO: 4, SEQ ID NO: 5 or SEO ID NO: 6.

5. The method according to any one of claims 1 to 4 wherein NTP analogues are used.

6. The method according to any one of claims 1 to 5 wherein the enzymatic separation of the RNA/antisense RNA duplex is carried out by a helicase.

7. The method according to any one of claims 1 to 6 wherein the primer is a heteropolymeric or homopolymeric RNA or DNA primer.

8. The method of claim 7 wherein the primer has a length of from 20 to 25 bases.

9. The method of claim 7 or 8 wherein the homopolymeric primer is a poly-U primer and the RNA template is cellular total RNA such that a specific amplification of the total cellular RNA occurs.

10. The method of claim 7 or 8 wherein the RNA template is cellular total RNA and the heteropolymeric primer hybridises specifically with a specific segment of viral RNA.

11. The method of claim 10 wherein the cellular total RNA is extracted from material of a patient before step (a).

12. The method of claim 11 wherein the material of a patient is selected from the group consisting of liquor, blood, plasma or body fluids.

13. A kit for the primer-dependent amplification of heterologous viral, eukaryotic or prokaryotic RNA, at least consisting of:
a. an RNA-dependent RNA polymerase as defined in any one of claims 1 to 4;
b. a suitable reaction buffer;
c. NTPs;
d. an enzyme capable of separating double-stranded RNA into single stranded RNA, preferably a helicase;
e. oligonucleotide primer;
f. optionally, RNAse inhibitor;
g. optionally, stop solution.

## Revendications

1. Procédé d'amplification *in vitro* d'ARN hétérologue viral, eucaryote ou procaryote, comprenant les étapes de :
a. fixation d'une ARN polymérase ARN-dépendante sur la matrice d'ARN en présence d'une amorce oligonucléotidique spécifique d'une séquence, qui s'hybride avec une portion de la matrice d'ARN,
b. transcription de la matrice d'ARN en ARN antisens, l'amorce étant prolongée par l'ARN polymérase ARN-dépendante correspondant à la séquence de la matrice d'ARN,
c. séparation du duplex ARN/ARN antisens en simples brins d'ARN par dénaturation thermique, dénaturation chimique et/ou grâce à une enzyme ayant la faculté de séparer l'ARN double brin en ARN simple brin,
d. le cas échéant répéter les étapes a. à c.,
dans lequel l'ARN polymérase ARN-dépendante présente une « conformation main droite » et la séquence d'acides aminés de l'ARN polymérase ARN-dépendante présente les portions de séquences suivantes :
a. XXDYS
b. GXPSG
c. YGDD
d. XXYGL
e. XXXXFLXRXX
avec la signification :
D : aspartate
Y : tyrosine
S : sérine
G : glycine
P : proline
L : leucine
F : phénylalanine
R : arginine
X : un acide aminé quelconque.

2. Procédé selon la revendication 1, dans lequel l'ARN polymérase ARN-dépendante est une ARN polymérase ARN-dépendante d'un virus de la famille des *Caliciviridae.*

3. Procédé selon la revendication 2, dans lequel l'ARN polymérase ARN-dépendante est une ARN polymérase ARN-dépendante d'un norovirus, sapovirus, vésivirus ou lagovirus.

4. Procédé selon la revendication 3, dans lequel l'ARN polymérase ARN-dépendante est une protéine selon SEQ ID N°1, SEQ ID N°2, SEQ ID N°3, SEQ ID N°4, SEQ ID N°5 ou SEQ ID N°6.

5. Procédé selon l'une des revendications 1 à 4, dans lequel des analogues de NTP sont mis en oeuvre.

6. Procédé selon l'une des revendications 1 à 5, dans lequel la séparation enzymatique du duplex ARN/ARN antisens se fait par une hélicase.

7. Procédé selon l'une des revendications 1 à 6, dans lequel l'amorce est une amorce d'ARN ou d'ADN hétéropolymérique ou homopolymérique.

8. Procédé selon la revendication 7, dans lequel l'amorce présente une longueur de 20 à 25 bases.

9. Procédé selon la revendication 7 ou 8, dans lequel l'amorce homopolymérique est une amorce d'ARN poly-U et la matrice d'ARN de l'ARN cellulaire entier, si bien qu'il se produit une amplification spécifique de l'ARNm cellulaire entier.

10. Procédé selon la revendication 7 ou 8, dans lequel la matrice d'ARN est de l'ARN cellulaire entier et l'amorce hétéropolymérique s'hybride spécifiquement avec une portion déterminée de l'ARN viral.

11. Procédé selon la revendication 10, dans lequel, avant l'étape a., l'ARN cellulaire entier est obtenu à partir d'une substance d'un patient.

12. Procédé selon la revendication 11, dans lequel la substance d'un patient est choisie dans le groupe constitué par le liquide céphalo-rachidien, le sang, le plasma ou les fluides corporels.

13. Kit d'amplification amorce-dépendante d'ARN hétérologue viral, eucaryote ou procaryote, se composant au moins de :
a. une ARN polymérase ARN-dépendante telle que définie dans l'une des revendications 1 à 4,
b. un tampon de réaction approprié,
c. des NTPs,
d. une enzyme ayant la faculté de séparer l'ARN double brin en simple brin, de préférence une hélicase,
e. une amorce oligonucléotidique,
f. le cas échéant un inhibiteur d'ARNase,
g. le cas échéant une solution d'arrêt.
